# EUROPEAN PATENT APPLICATION

(11) **EP 1 271 384 A1**
(43) Date of publication of application: **02.01.2003**
(21) Application number: 01115825.0
(22) Date of filing: 28.06.2001
(51) Int. Cl.: G06F 19/00

(54) **System and method for assisting in diagnosis, therapy and/or monitoring of a funtional lung disease**

(71) Applicant: Boehringer Ingelheim International GmbH, 55218 Ingelheim am Rhein (DE); Erich Jaeger GmbH, 97204 Höchberg (DE)
(72) Inventor: Schau, Brigitte, 37124 Rosdorf (DE); Schülke, Hans-Joachim, 97320 Mainstockheim (DE); Reinstädtler, Jürgen, 97204 Höchberg (DE)
(74) Representative: Betten & Resch

(57) **Abstract**

A computer-implemented method of assisting in diagnosis, therapy, follow-up and/or monitoring of a functional lung disease comprises entering patient demographic data, determining predicted lung function parameters from these demographic data, obtaining lung function measurement data from a spriometry measurement of the patient's lung function, and determining a diagnosis proposal dependent on the demographic data, the predicted and the measured lung function parameters of the patient. The present invention gives a physician, a health manager or the patient himself/herself assistance in the correct diagnosis of a functional lung disease and checking or evaluating an earlier diagnosis and treatment strategies based thereon. The invention helps to find suitable treatment strategies for an individual patient and also allows an early detection of a functional lung disease even if the patient does not yet show corresponding symptoms.

## Description

The present invention relates to a computer system, computer-implemented method and computer program assisting a physician, a case manager or the patient himself/herself in diagnosis/therapy/follow-up/monitoring functional lung diseases like asthma or chronic obstructive pulmonary disease (COPD).

### DESCRIPTION OF RELATED ART

Diagnosis and treatment of pulmonary diseases is based on published medical guidelines like for example of the American Thoracic Society, Standards for the Diagnosis and Care of Patients with Chronic Obstructive Pulmonary disease, Am I Respir/Crit Care Med. Vol. 52, pages S77 - S120, 1995, Siafakas, N.M., Vermeire, P., Pride, N.B., ERS Consensus Statement: Optimal Assessment and Management of Chronic Obstructive Pulmonary Disease (COPD), Eur Respir J, 8, 1398 - 1420, 1995; National Heart, Lung and Blood Institute, 1995. Global Initiative for Asthma. U.S. Government Printing Office, Washington, DC. Publication No. (NIH) 95-3659. Based on the patient's disease history, patient's symptoms and measurement results like spirometry/lung function measurements using a spirometer the physician makes his/her diagnosis, discusses the same with the patient and enters it into the patient's records. Depending on the diagnosis the physician checks whether or not a medication is appropriate and when the patient is already on a treatment regimen proposes, if necessary, alternative therapies. The success of the therapy is then reviewed during the next visits of the patient.

For making his/her diagnosis, the physician has to review the patient records and, if respiratory measurements are carried out and have to be interpreted, he/she frequently has to look up in detail the interpretation of the measurement values in the relevant guidelines as well as the implication of these results with regard to chronic obstructive lung disease. This process is complicated and time-consuming.

### SUMMARY OF THE INVENTION

It is therefore an object of the present invention to provide the physician case manager or patient with assistance for diagnosis, therapy, follow-up and/or monitoring of pulmonary diseases.

The present invention provides a computer-implemented system for assisting in the diagnosis and/or therapy of a functional lung disease comprising: a data entry unit for entering patient demographic data; a storage unit for storing the patient demographic data; an input unit for receiving data from a spirometry measurement instrument; a calculating unit for determining predicted lung function parameters derived from the patient demographic data, for processing the received spirometry measurement data, and for determining a diagnosis proposal based on the patient demographic data, predicted lung function parameters, spirometry measurement results and underlying medical guidelines.

The present invention further provides a computer-implemented method of assisting in the diagnosis and/or therapy of a functional lung disease, the method comprising inputting patient demographic data, determining predicted lung function parameters from the patient demographic data, inputting measurement data of spirometry measurements of the patients's lung function, and determining a diagnosis proposal based on the demographic data, the calculated predicted lung function parameters and the received spirometry measurement results. The present invention thus can assist a physician, case manager or the patient himself/herself in diagnosis, follow-up, monitoring and/or therapy of pulmonary diseases like in particular asthma or COPD. The spirometry measurements for obtaining lung function data may be obtained by an analog or preferably by a digital spirometer. The measurement data are transferred through a suitable communication line to the computer system and may be stored in suitable storage device. Alternatively the input of the spirometry results may be performed manually.

The measured lung function parameters may include FVC and FEV1 as well as other parameters like FEV6, PEF, FEF25, FEF50, FEF75 or MMEF.

Preferably the reproducability of the spirometry measurement results is checked. A sufficient degree of reproducability is e. g. assumed if three consecutive FCV measurements do not vary by more than 10 ml to 600 ml, preferably not more than 200 ml. A positive reproducability check may be indicated by an optical or acoustical signal.

The computer system according to the present invention allows storing varying treatment guidelines of professional associations, health care providers or health insurance companies of different countries which can easily be updated and used for determining the diagnosis proposal.

The calculated predicted lung function data and the measurement results can preferably be displayed on a display screen, wherein different colours may be used for different measurements.

According to a particular embodiment the present invention comprises a reversability testing procedure including a first FEV1 measurement and a second FEV1 measurement after administration of a bronchodilator medication and a waiting period depending on the bronchodilator. If the second FEV1 data shows a certain amount of improvement, that is the bronchodilator shows a positive effect and the criteria for reversibility are met according to guidelines/current medical practice, an asthma diagnosis is proposed.

According to a further embodiment the present invention includes a severity grading of a obstructive pulmonary disease of the patient. For determining the severity grading for COPD a measured FEV1 value is compared with the corresponding predicted value. For determining the severity in the case of asthma further factors are taken into account including patient symptoms and the current medication.

According to a still further embodiment the present invention includes a therapy evaluation process comprising obtaining a control score, which is calculated by comparing an FEV1 measurement result with the individual previous best FEV1 result of the same patient and further taking into account the factors like effort tolerance in the case of COPD and FEV1 variability, symptoms and medication in the case of asthma.

The invention further provides a computer program product according to claim 65.

The present invention has the advantage that the physician (or case manager) gets reliable and verifiable assistance for diagnosis and therapy of patients with pulmonary diseases. All relevant data of a patient can be stored and are available upon request. With the system of the present invention considerable time savings can be realized for the physician at any follow-up visit. The graphical illustration of the lung function measurement results improves understanding of the disease by the patient.

Moreover, the physician can easily adapt his/her diagnosis and therapy to the scientific development by using the update according to the latest guidelines of professional associations and organizations. The present invention facilitates a correct diagnosis of pulmonary diseases, in particular asthma and COPD and reduces the risk of erroneous diagnosis.

Furthermore, the present invention helps to reach an early diagnosis of a functional lung disease even if the patient does not yet show symptoms and thus makes an early therapy possible.

The present invention and further features, objects and advantages thereof will be more readily apparent from the following detailed description of particular embodiments thereof, taken in conjunction with the accompanying drawings.

### BRIEF DESCRIPTION OF THE DRAWINGS

Fig. 1 is a flow chart schematically illustrating method steps of an embodiment of the present invention;
Fig. 2 is a flow chart illustrating the reversibility test according to a particular embodiment of the present invention;
Fig. 3 is a schematic illustration of an exemplary screen display of a particular embodiment of the system according to the invention;
Fig. 4 is a schematic illustration of a hardware configuration to which the present invention may be applied;
Fig. 5 is a flow chart illustrating the COPD severity grading process according to a particular embodiment of the present invention;
Fig. 6 is a flow chart illustrating the asthma severity grading process according to a particular embodiment of the present invention;
Fig. 7 is a flow chart illustrating the therapy evaluation process according to a further particular embodiment of the present invention;
Fig. 8 shows a table used for determining the COPD severity grading;
Fig. 9 shows an example of a matrix used for determining the asthma severity grading;
Fig. 10 shows an example of a table used for obtaining a COPD control score; and
Fig. 11 shows an example of a table used to obtain an asthma control score.

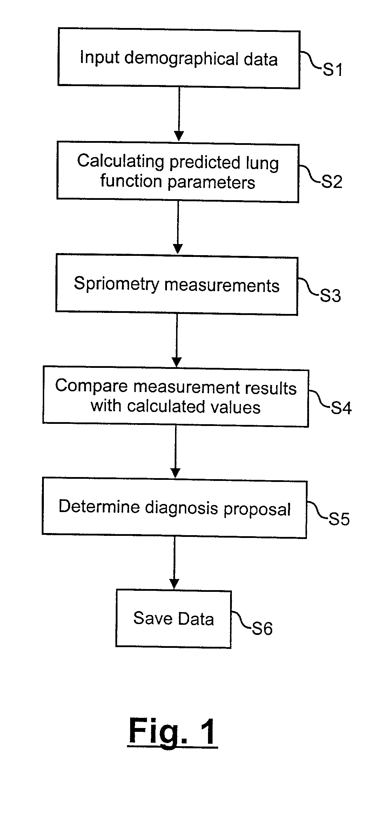

Fig. 1 is a flow chart illustrating the method steps of a first embodiment of the computer-implemented method of assisting a physician (or case manager) in the diagnosis of a pulmonary disease. In method step S1 patient demographic data are inputted if the patient is diagnosed for the first time with this system. In other cases the patient name or appropriate identification data are input for retrieving the patient demographic data including age, sex, and race. Based on these data and medical guidelines for the diagnosis of pulmonary diseases stored in a computer memory the predicted lung function parameters of the patient are calculated in method step S2. Preferably these are displayed on a display screen as will be discussed in detail below.

In the next method step S3 spirometry measurements are carried out using an appropriate spirometer as for example the PM2 spirometer of the manufacturer Jaeger. With this device lung function parameters as the forced expiratory volume in one second (FEV1) and the forced vital capacity (FVC) or the forced expiratory volume in six seconds (FEV6) which can be used instead of FCV of the patient can be measured.

Other functional lung parameters which may be measured include the Peak Expiratory Flow (PEF), the Forced Expiratory Flow at a Certain Percentage of the Forced Vital Capacity (e. g. FEF25, FEF50 and FEF75 being the Forced Expiratory Flow at 25%, 50% and 75%, respectively of the Forced Vital Capacity) and the mean maximum expiratory flow (MMEF).

It is then checked if the quality of the measurement for example the reproducibility is sufficient (will be explained in detail later) and subsequently the measurement results are compared with the predicted lung function parameters of the patient. Based on these data a diagnosis proposal is determined in step S4 and displayed on the display screen for review by the physician. It is up to the physician to then accept the diagnosis proposal or to override the system with a different diagnosis. After finishing the diagnosis all patient-related data including measurement results are saved and can be retrieved at any time.

### Reversibility Test

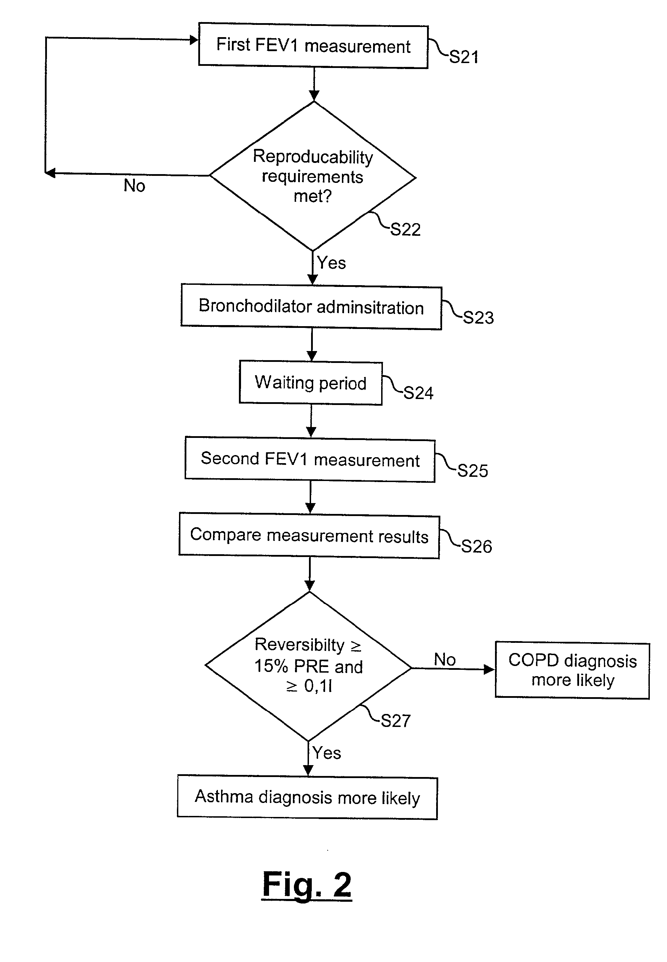

Fig. 2 is a flow chart explaining in more detail the reversibility test as one important criterion distinguishing between an asthma diagnosis and a COPD diagnosis.

After retrieving data and calculating the predicted lung function parameters (steps S1 and S2 of Fig. 1) a first FEV1 measurement is carried out in step S21. If the reproducability is satisfactory the patient is administered a bronchiodilator in step S23. After a waiting time depending on the medication, a second FEV1 measurement is carried out (step S24). The results of the two measurements are then compared with each other and if the reversibility is for example at least 15% of the theoretically predicted FEV1 of the patient and is at least 0.2 litres, i.e. the FEV1 result of the second measurement is at least 0.2 litres and 15% of the theoretical value better than the corresponding result of the first measurement, an asthma diagnosis is more likely since the patient responds to bronchiodilator treatment. If the reversibility is below these values a COPD diagnosis is more likely. The diagnosis then proceeds on these assumptions. Other threshold values may be used for the reversability test, preferably between 5% and 25% and 10 ml and 600 ml, respectively.

### Screen Display

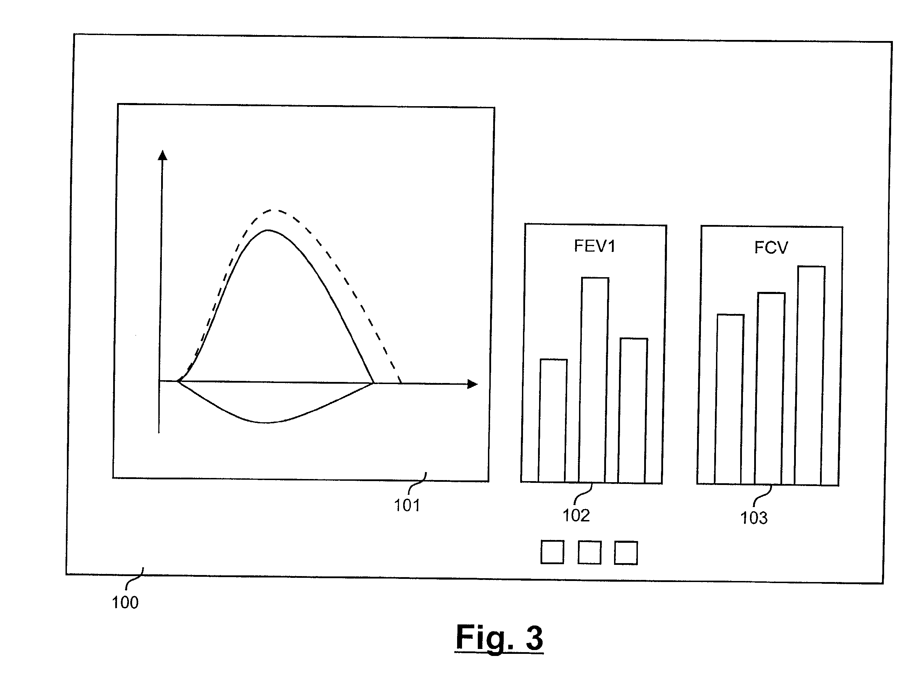

Fig. 3 is a schematic view of an exemplary screen display of the system according to an embodiment of the present invention. On the screen, designated with reference numeral 100, the lung function parameters are displayed on a first window 101. On the X axis the inspiratory/expiratory volume in litres and on the Y axis the airflow in litres per second is depicted. The dotted line represents the expected or predicted values whereas the measurement data are represented by the solid curves. Multiple measurements may be represented by different colours. On additional windows 102, 103 the measurement results of FEV1 and FVC are additionally displayed as column diagrams.

The reproducability of a respective measurement is preferably represented by a colour signal on the bottom of the screen. If the deviation between subsequent measurements (for example three tests total) exceeds 0.4 litres a red signal appears, if the deviation is between 0.2 and 0.4 litres a yellow signal appears and a green signal if the deviation between the measurements is smaller than 0.2 litres. For determining the reproducability of the measurement results other threshold values may also be applied.

### Hardware configuration

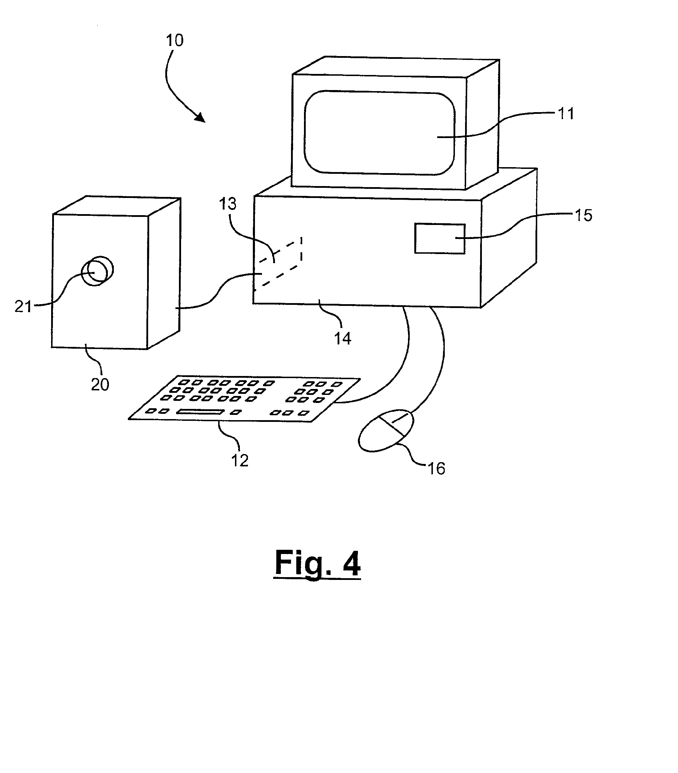

Fig. 4 shows an exemplary hardware configuration to which the present invention may be applied.

A computer like a desktop computer 10 comprises a central processing unit (CPU 14), a storage device like a hard disk, CD-Rom and/or DVD-Rom, a display screen 11 and input devices like a keyboard 12, a mouse 16 or speech input means (not shown). An interface 13 like an appropriate PC-card allows receiving data from a spirometer 20, which may be an analog device or preferably a digital instrument. The spirometer 20 preferably comprises a detachable mouth piece 21.

The computer system 10 may also be a laptop computer, a personal digital assistant or a terminal device of an interconnected network of a plurality of computers.

### Severity grading process

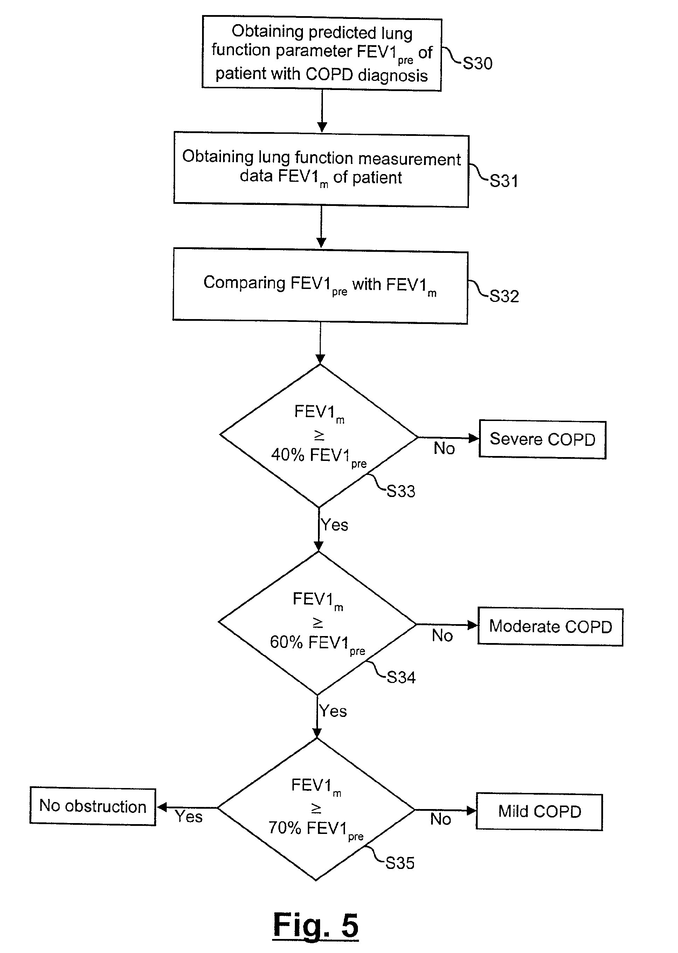

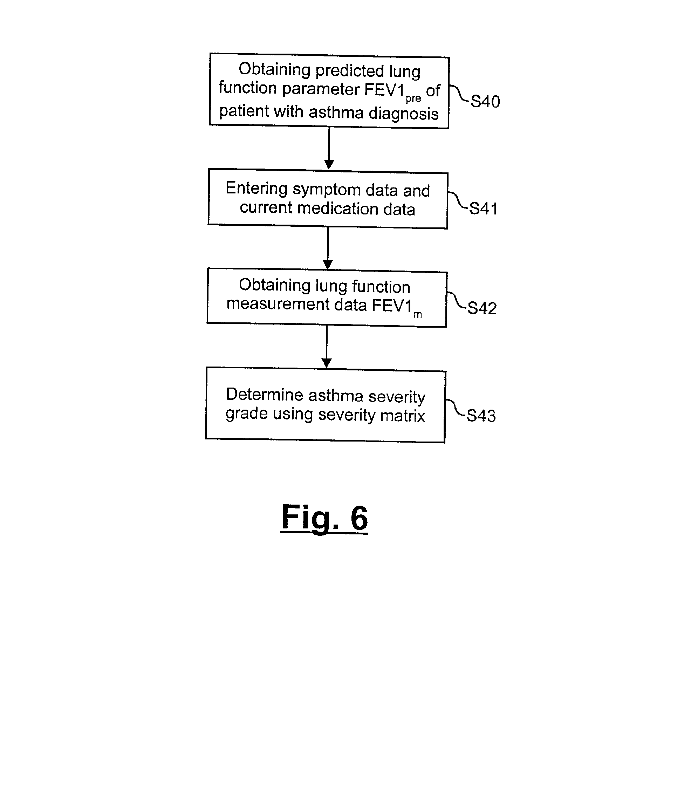

The flowcharts of Fig. 5 and 6 schematically illustrate the severity grading procedure for COPD and asthma, respectively. For other lung diseases similar processes may be used. The severity grading process is based on underlying guidelines.

In method step S30 the predicted lung function parameter FEV1ₚᵣₑ of a patient with a COPD diagnosis is obtained from the demographic data of the patient. Preferably the severity grading process is carried out subsequently to the reversability test explained before with reference to Fig. 2. Based on the diagnosis proposal obtained in the reversability test the severity grading can be carried out for COPD or for asthma. It is, however, also possible to perform the severity grading procedure without the reversibility test if the diagnosis is known from earlier consultation.

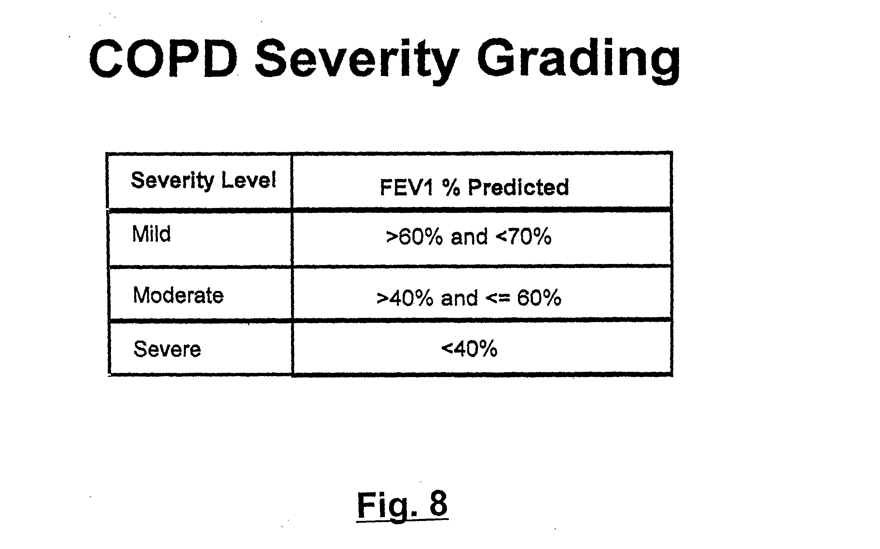

In the next step S31 a spirometry measurement of the patient is carried out (or results of an earlier spriometry test retrieved) and the measured parameter FEV1ₘ is obtained. In the subsequent step S32 the two values FEV1ₚᵣₑ and FEV1ₘ are compared with each other and depending on the result of the comparison different severity gradings, no obstruction detected, mild COPD, moderate COPD and severe COPD are obtained (method steps S33 to S35). The COPD severity grading is further illustrated in the table of Fig. 8. If the measured FEV1 value is at least 70% of the predicted value the patient is regarded as not suffering from COPD. A measurement value of between 60% and 70% means mild COPD, between 40% and 60% moderate COPD and a value of below 40% severe COPD. The values for the severity grading depend on the underlying guidelines and may be subject to change.

The flowchart of Fig. 6 illustrates the severity grading process in the case of an asthma patient.

In step S40 the predicted lung function parameter data FEV1ₚᵣₑ of the patient with asthma diagnosis are obtained from a storage device or calculated from the demographic data. As in the case of COPD an asthma diagnosis may be obtained from an earlier reversability measurement.

In method step S41 patient symptom data like exacerbations and medication data like the use of a bronchodilator or corticosteroid are entered. Then, in the next step S42 measured lung function parameter FEV1ₘ is obtained.

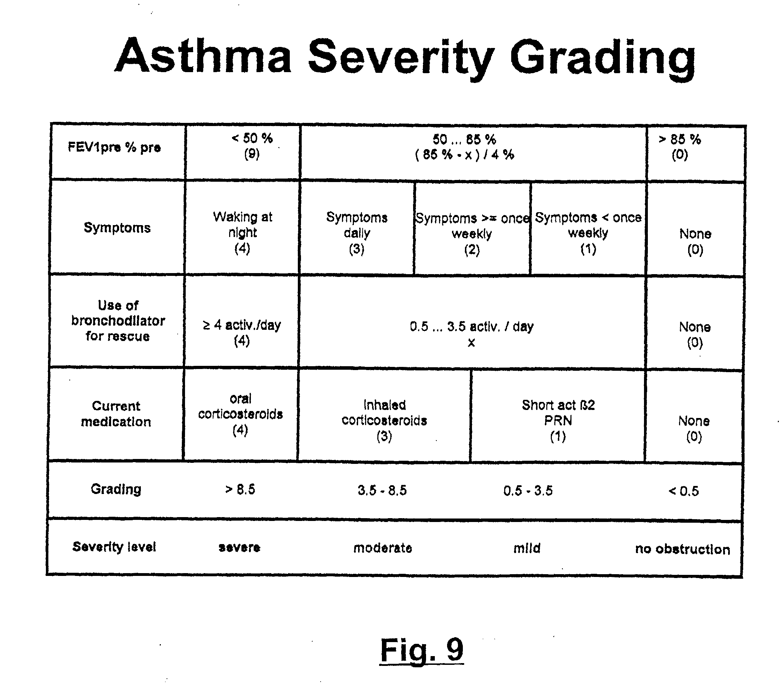

In the subsequent step S43 the asthma severity grade is determined using the asthma severity grading table shown in Fig. 9. The severity grading depends on a total of four factors, i. e. the measured FEV1 value as a percentage of the predicted value, the severity of the symptoms of the patient, the frequency of the use of bronchodilator rescue medication and the current corticoid medication by the asthmatic patient. Each of these factors is reflected by corresponding severity values contributing to the total severity grading value. The lung function parameters are assigned values between 0 and 9 as shown in the first line of the table. An FEV1 of 85% corresponds to a value of 0 and a measurement result of 50% to a value of 9. The patient symptoms are assigned values between 0 and 4 as shown in the second row of the table and the frequency of the daily use of bronchodilator rescue medication contributes to the overall grading with values between 0 and 4. In the same way the use of oral corticosteroids means a value of 4, the use of inhaled corticosteroids a value of 3, and the use of β2 PRN a value of 1. The values of the three lines with the highest score are added to form the overall grading. A grading of higher than 8.5 means a severe asthma, a value between 3.5 and 8.5 moderate asthma, a value of 0.5 to 3.5 mild asthma and a value of less than 0.5 no obstruction. The parameters as well as the number may be subject to change depending on the underlying guidelines.

### Therapy evaluation process

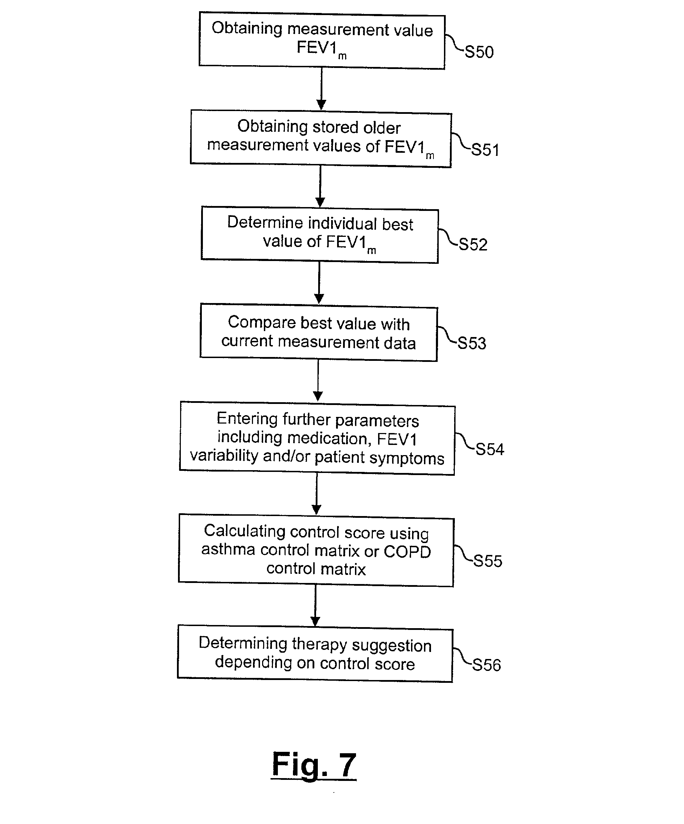

The flowchart of Fig. 7 illustrates the steps of the therapy evaluation procedure according to an embodiment of the present invention. In the first step FEV1 measurement data are obtained by a spirometric measurement. Then, in subsequent method step S51 stored data of previous FEV1 measurements are retrieved and the individual best FEV1 measurement data of this patient are determined in method step S52. This personal best value is compared with the new measurement data forming one factor for the determination of the asthma or COPD control score. Other factors are the effort tolerance in the case of COPD and, in the case of asthma, the FEV1 variability, patient symptoms and the use of β2 adrenalin rescue medication.

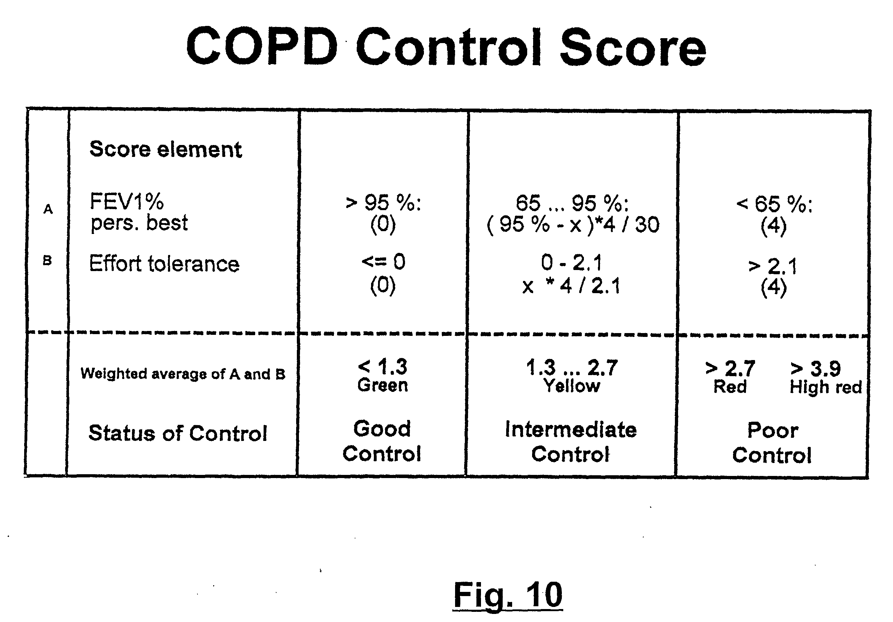

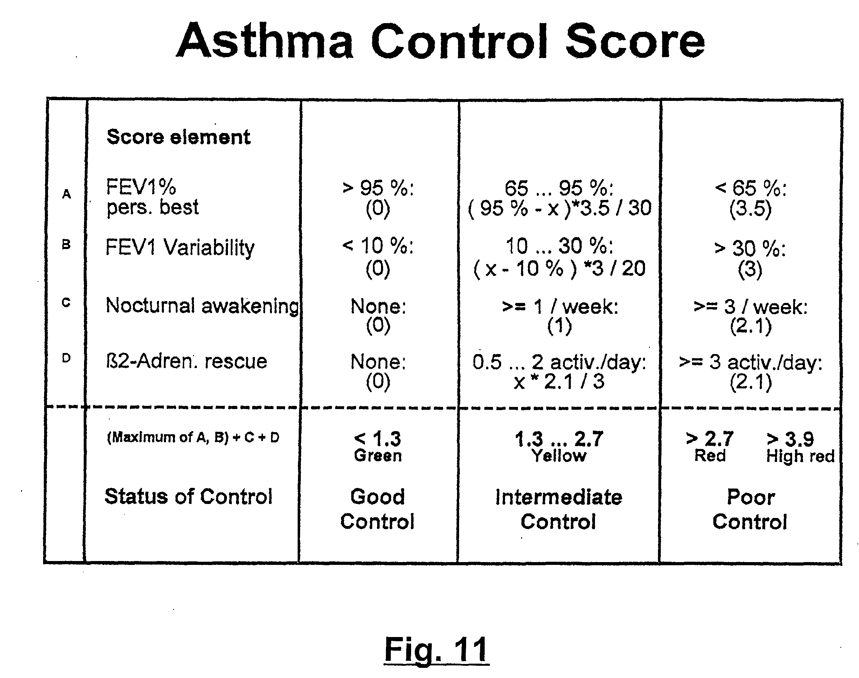

In the following method step S55 the asthma control score or COPD control score is calculated using the respective control score table depicted in Fig. 10 and 11, respectively.

For determining the COPD control score the FEV1 measurement value as percentage of the individual best value of the patient is taken into account with assigning a value of 0 for FEV1 greater than 95% and a value of 4 for FEV1 below 65% with respect to the personal best value. The effort tolerance is also assigned values between 0 and 4 based on empiric data. The weighted average of these two values A and B (see Fig. 10) then forms the COPD control score. A value lower than 1.3 means a good control, a value of between 1.3 and 2.7 an intermediate control and values over 2.7 a poor control. Based on the control score amended therapy suggestions are determined in method step S56.

The calculation of the asthma control score is shown in the table of Fig. 11. Similar like in the case of COPD the FEV1 value as percentage of the individual best value of the patient contributes as factor A between 0 and 3.5. The variability of the FEV1 value, that means how much the measurement results vary from one measurement to another, is taken into account as a factor B. Nocturnal awakening symptoms and the use of β2-adrenalin rescue medication is then translated into factors C and D. At the end the maximum of factors A or B is added to factors C and D to form the asthma control score. As in the case of COPD a score of less than 1.3 means a good control, a value of between 1.3 and 2.7 an intermediate control and higher values a poor control. In the latter case an alternative therapy suggestion is proposed. The parameters and numbers may be subject to change depending on the relevant guidelines.

## Claims

1. A computer-implemented system for assisting in the diagnosis, therapy, monitoring and/or follow-up of a functional lung disease comprising:
- a data entry unit (12) for entering patient demographic data,
- a storage unit for storing the patient demographic data,
- an input unit (13) for receiving data from a spirometer,
- a calculating unit (14) for determining predicted lung function parameters derived from the patient demographic data, for processing the received spirometry measurement data, and for determining a diagnosis proposal based on the patient demographic data, predicted lung function parameters, spirometry measurement results and underlying medical guidelines.

2. The computer system of claim 1, wherein the storage unit (15) is adapted for storing the received spirometry measurement data.

3. The computer system of claim 1 or 2, wherein the further storage unit (15) is adapted for storing further information about the patient.

4. The computer system of one of claims 1 to 3, wherein the input unit (13) is adapted for receiving data from an analog spirometry measurement instrument.

5. The computer system of one of claims 1 to 3, wherein the input unit (13) is adapted for receiving data from a digital spirometry measurement instrument.

6. The computer system of one of claims 1 to 5, wherein the spirometry measurement data includes measurement data of the parameters FVC (Forced Vital Capacity) and FEV1 (Forced Expiratory Volume in one Second).

7. The computer system of one of claims 1 to 6, wherein the spirometry measurement data include measurement results of the parameters PEF (Peak Expiratory Flow), FEF (Forced Expiratory Flow at a predetermined percentage of the Forced Vital Capacity) and/or MMEF (Mean Maximum Expiratory Flow).

8. The computer system of one of claims 1 to 7, wherein the calculating unit (14) is adapted for testing the reproducability of the received spirometry measurement data.

9. The computer system of claim 8, wherein a sufficient degree of reproducability is assumed if three subsequent FVC or FEV1 measurement results do not vary by more than a predetermined value between 10 ml and 600 ml, preferably about 200 ml.

10. The computer system of claim 8 or 9, comprising means for indicating the result of the reproducability test by an optical or acoustical signal.

11. The computer system of one of claims 8 to 10, comprising a user interface for removing individual measurement results.

12. The computer system of one of claims 1 to 11, comprising a storage unit for storing varying guidelines and/or suggestions from different countries for the diagnosis/treatment and follow-up of functional lung diseases.

13. The computer system of one of claims 1 to 12, comprising a display unit (11) for graphically displaying the spirometry measurement data and/or the calculated predicted lung function parameters.

14. The computer system of claim 13, wherein the display unit (11) is adapted for further displaying animated pictures for motivating patients to attend the lung function measurements.

15. The computer system of claim 13 or 14, wherein the graphical representation of the measurement data and predicted lung function parameter data comprising displaying the inspiratory and expiratory volume on the X axis and the airflow on the Y axis.

16. The computer system of one of claims 1 to 15, wherein the storage unit (15) is adapted for storing patient disease history data including exacerbations, smoking data, allergies, patient symptoms and current medication data.

17. The computer system of one of claims 1 to 16, wherein the display unit (11) is adapted for displaying the results of respective spirometry measurements in different colours.

18. The computer system of one of claims 1 to 17, wherein the calculating unit (14) is adapted for determining a diagnosis proposal taking into account patient disease history data and/or disease treatment guidelines of professional associations, insurance companies and/or health care organizations.

19. The computer system of one of claims 1 to 18, wherein the calculating unit (14) is adapted for carrying out a reversibility test including a first measurement of FEV1, a second measurement of FEV1 after administration of a bronchodilatator and comparing the results of the first and second FEV1 measurements.

20. The computer system of claim 19, wherein an asthma diagnosis is suggested if the reversability of FEV1 exceeds a predetermined percentage of the predicted FEV1 volume of that patient and exceeds a predetermined threshold volume and/or patient history suggestive of asthma with irreversible component.

21. The computer system of claim 20, wherein the predetermined percentage is between 5% and 25%, preferably about 15%, and the predetermined threshold volume is between 10 ml and 500 ml, preferably about 100 ml.

22. The computer system of one of claims 19 to 21, wherein the time period between administration of bronchodilator medication and the second FEV1 measurement has a fixed duration depending on a drug used and on current medical practice in specific country.

23. The computer system of claim 22, wherein the computer system is adapted for processing data of other patients during the waiting period and the display unit (11) is adapted for displaying the remaining waiting period for the second FEV1 measurement.

24. The computer system of one of claims 1 to 23, wherein a calculating unit (14) is adapted for carrying out a severity grading process of the lung disease based on the calculated predicted lung function parameters and the received spirometry measurement results.

25. The computer system of claim 24, wherein the severity grading process includes comparing the predicted FEV1 value with the measured FEV1 value.

26. The computer system of claim 25, wherein severe COPD is suggested if the measured FEV1 value is lower than 40% of the predicted value, moderate COPD if the measured FEV1 value is between 40% and 60% of the predicted value and mild COPD if the measured FEV1 value is between 60% and 70% of the predicted value depending on guidelines implemented.

27. The computer system of claim 25, wherein the severity grading process determines severe asthma if the measured FEV1 value is lower than 50% of the predicted value and determines mild to moderate asthma if the measured FEV1 value is between 50% and 85% of the predicted value.

28. The computer system of claim 27, wherein the severity grading process includes taking account other parameters including patient symptoms and current medication data.

29. The computer system of one of claims 1 to 28, wherein the calculation unit (14) is adapted for carrying out a therapy evaluation process comprising calculating a control score based on the predicted lung function parameters derived from the patient demographic data, the spirometry measurement data and patient history data.

30. The computer system of claim 29, wherein the calculation of the control score includes comparing the FEV1 measurement results with previous measurement results of that patient.

31. The computer system of claim 29 or 30, wherein the calculation of the control score takes into account the variabiliy of FEV1 measurement data, patient medication data and/or patient symptom data.

32. A computer-implemented method of assisting in the diagnosis and/or therapy of a functional lung disease, the method comprising:
- inputting patient demographic data,
- determining predicted lung function parameters from the patient demographic data,
- inputting of spirometry measurements data of the patient's lung function, and
- determining a diagnosis proposal based on the demographic data, the calculated predicted lung function parameters and the received spirometry measurement results.

33. The method of claim 32 comprising storing spirometry measurement data on a storage unit (15) for comparison with later measurements.

34. The method of claim 32 or 33 comprising storing on a storage unit in a patient file text data including a patient history, patient symptoms and/or medication data input by a physician, health manager or the patient himself.

35. The method of one of claims 32 to 34 wherein the measurements of the patient's lung function are carried out using a digital spirometry measurement instrument.

36. The method of one of claims 32 to 34 wherein the measurement data of the patient's lung function are obtained through analog measurement.

37. The method of one of claims 32 to 36, wherein the measured lung function parameters include FVC and FEV1.

38. The method of one of claims 32 to 37, wherein the measured lung function parameters include PEF, MMEF and/or FEF λ, wherein λ is a percentage of the forced vital capacity.

39. The method of one of claims 32 to 38 comprising detecting the reproducability of the lung function measurement data.

40. The method of claim 39, wherein a sufficient degree of reproducability is assumed if three subsequent FVC or FEV1 measurement results do not vary by more than a predetermined value between 10 ml and 600 ml, preferably about 200 ml.

41. The method of claim 39 or 40 comprising indicating a sufficient degree of reproducability by an optical or acoustical signal.

42. The method of one of claims 32 to 41 comprising excluding at least one individual measurement result from further processing.

43. The method of one of claims 32 to 42, wherein the determination of the diagnosis proposal is based on diagnosis guidelines stored in storage device.

44. The method of claim 43 wherein the diagnosis guidelines include the guidelines of different medical and technical professional associations, insurance companies and health care providers from various countries.

45. The method of one of claims 33 to 44 including displaying the measured and calculated predicted lung function data graphically on a display screen.

46. The method of claim 45 including further displaying animated pictures for motivating patients attending the spirometry measurements.

47. The method of claim 45 or 46, wherein the graphical representation of the measurement results comprises displaying the inspiratory and expiratory volume on the X axis and the airflow on the Y axis.

48. The method of one of claims 45 to 47 comprising displaying the results of different lung function measurements in different colours.

49. The method of one of claims 32 to 48 comprising a reversibility testing process including a first FEV1 measurement and a second FEV1 measurement after administration of bronchodilator medication and comparing the two measurement results.

50. The method of claim 49, wherein an asthma diagnosis is suggested if the reversibility of FEV1 exceeds a predetermined percentage of predicted FEV1 volume of that patient and exceeds a predetermined threshold volume.

51. The method of claim 50, wherein the predetermined percentage is between 5% and 25%, preferably about 15%, and the predetermined threshold volume is between 10 ml and 500 ml, preferably about 100 ml.

52. The method of claim 49, 50 or 51, wherein the time period between administration of bronchodilator medication and the second FEV1 measurement has a duration depending on medication supplied.

53. The method of claim 52 comprising displaying a remaining waiting period and processing data of other patients during the waiting period.

54. The method of one of claims 32 to 53 comprising carrying out a severity grading process of the lung disease based on the predicted lung function parameters and the obtained lung function measurement results.

55. The method of claim 54 wherein the severity grading process includes comparing the predicted FEV1 value of a patient with the measured FEV1 value.

56. The method of claim 55, wherein severe COPD is suggested if the measured FEV1 value is lower than 40% of the predicted value, moderate COPD if the measured FEV1 value is between 40% and 60% of the predicted value and mild COPD if the measured FEV1 value is between 60% and 70% of the predicted value depending on guidelines implemented.

57. The method of claim 54, wherein the severity grading process determines severe asthma if the measured FEV1 value is lower that 50% of the predicted value and determines mild to moderate asthma if the measured FEV1 value is between 50% and 85% of the predicted value.

58. The method of claim 57, wherein the severity grading process includes taking into account other parameters including patient symptoms and current medication data.

59. The method of one of claims 32 to 58 comprising a therapy evaluation process including determining a control score dependent on a comparison of current lung function measurement results with earlier lung function measurement results.

60. The method of claim 59 wherein the earlier lung function measurement results are the individual best results of the respective patient.

61. The method of claim 59 or 60 wherein the control score further depends on the present medication of the patient, input symptoms of the patient and/or a variability of the lung function measurement results.

62. The method of one of claims 59 to 61 comprising determining an alternative therapy suggestion if the control score is below a predetermined value.

63. A method of assisting in therapy of a functional lung disease comprising:
- inputting patient demographic data,
- inputting patient history data including present treatment data,
- determining predicted lung function parameters from the patient demographic data,
- obtaining lung function measurement data,
- determining a diagnosis proposal based on the demographic data, predicted lung function parameters and measured lung function parameters, and
- comparing the diagnosis proposal with the present treatment data and, based on the comparison result, determining a change of treatment strategy.

64. A computer program comprising computer-executable program code for carrying out a method of one of claims 32 to 63.

65. A computer program product comprising computer-executable program code for carrying out a method of assisting in diagnosis and/or therapy of a functional lung disease, the method comprising:
- inputting patient demographic data,
- determining predicted lung function parameters from the patient demographic data,
- inputting measurement data of spirometry measurements of the patient's lung function,
- determining a diagnosis proposal based on the demographic data, the calculated predicted lung function parameters and the received spirometry measurement results, and
- optionally comparing the diagnosis proposal with the present treatment data and, based on the comparison result, determining a change of treatment strategy.
